# EUROPEAN PATENT APPLICATION

(11) **EP 4 159 240 A1**
(43) Date of publication of application: **05.04.2023**
(21) Application number: 21818496.8
(22) Date of filing: 28.05.2021
(51) Int. Cl.: A61K 45/00, A61P 3/04, A61K 9/00, A61K 36/00, A61K 47/36, A61K 38/46, A61M 37/00, A61K 31/047, A61K 31/165, A61K 31/198, A61K 31/205, A61K 31/522, A61K 31/575, A61K 31/685, A61K 31/7076

(54) **BEAUTY MICRONEEDLE ARRAY**

(30) Priority: 01.06.2020 JP 2020095712
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); KONDOU, Naoko, Kyoto-shi, Kyoto 601-8014 (JP); TANAKA, Hiroshi, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2021/020330
(87) International publication number: WO 2021/246301

(57) **Abstract**

Provided is a new means for administering a valuable substance having a fat dissolving action. A microneedle preparation including at least one of a lipolytic agent, a fat dissolving agent, or a fat metabolism promoting agent, the microneedle preparation being intended for fat reduction and/or partial slenderizing and/or skin tightening, and a microneedle preparation including a microneedle array containing a water-soluble polymer as a base and one or more selected from the group consisting of a lipase, a phosphatidylcholine, and deoxycholic acid.

## Description

### TECHNICAL FIELD

The present invention relates to a microneedle array intended to decompose and dissolve subcutaneous fat. The present invention specifically relates to the technical field of microneedles containing, as a main component, a fat dissolving agent or the like as a valuable component.

### BACKGROUND ART

Subcutaneous fat accumulation is associated with dysfunction of adipocytes. In adipocytes, accumulated triglycerides decompose enzymatically and break down into fatty acids, glycerol and/or glycerol esters. For various reasons, accumulation of triglycerides occurs in adipocytes, resulting in subcutaneous fat accumulation. This results in overweight and therapeutic and cosmetic problems.

For obesity having an excessive fat layer among subcutaneous fat accumulation and obesity, a method of directly and subcutaneously injecting a composition for removing subcutaneous fat accumulation is adopted. In this method, phosphatidylcholine preparations typified by Lipostabil manufactured by Sanofi-Aventis (Patent Documents 1 and 2) are used. There is also a known method of subcutaneously injecting deoxycholic acid. However, this method involves pain and swelling at the time of treatment and is regarded as problematic in terms of safety. It can be said that this method has a high risk. In addition, in the case of injection, it is necessary to shake the injection needle subcutaneously with respect to a certain area. Further, the degree of fat dissolving varies because of unevenly injected liquids, which causes bumpy skin.

### PRIOR ART DOCUMENT

### PATENT DOCUMENTS

Patent Document 1: JP 2007-509085 A
Patent Document 2: JP 2007-515439 A

### SUMMARY OF THE INVENTION

### PROBLEMS TO BE SOLVED BY THE INVENTION

An object of the present invention is to provide a new means for administering a valuable substance having a fat dissolving action.

### MEANS FOR SOLVING THE PROBLEM

Microneedles penetrate stratum corneum which interrupts absorption of fat dissolving agents. An effect of easily diffusing fat dissolving agents into subcutaneous tissue can be expected with microneedles. In particular, subcutaneous fat of an obese site is dissolved with microneedles, resulting in a partially slenderized body, a reduced eye bag, or a small face line. A tightening effect due to dissolving of subcutaneous fat at sagging parts can also be expected. Unlike injection, microneedle patches can provide care for a large area in one application. Since there has been no microneedle preparation intended for fat dissolving, there has been no knowledge about the concentrations of valuable substances, the frequency of skin application, and the like in forming the valuable substances described above into microneedles. Combination of valuable substances and how to secure the stability and skin migration property of the preparation are important issues. The inventors of the present invention have solved such problems, succeeded in producing a microneedle preparation containing a valuable substance having a fat dissolving action, and completed the present invention.

The present invention is as follows.
[1] A microneedle preparation including at least one of a lipolytic agent, a fat dissolving agent, or a fat metabolism promoting agent, the microneedle preparation being intended for fat reduction and/or partial slenderizing and/or skin tightening.
[2] A microneedle preparation including 0.1 mass% or more of one or more components that act on fat reduction selected from the group consisting of a lipolytic agent, a fat dissolving agent, and a fat metabolism promoting agent, the microneedle preparation being intended for fat reduction and/or partial slenderizing and/or skin tightening.
[3] The microneedle preparation according to [1] or [2], wherein the lipolytic agent is a lipase.
[4] The microneedle preparation according to [3], including a lipase, a Mg salt, and a Ca salt.
[5] The microneedle preparation according to [1] or [2], wherein the fat dissolving agent is one or more selected from the group consisting of a phosphatidylcholine, adenosine triphosphate and a sodium salt thereof, and deoxycholic acid.
[6] The microneedle preparation according to [1] or [2], wherein the fat metabolism promoting agent is one or more selected from the group consisting of an amino acid, L-carnitine, inositol, capsaicin, and caffeine.
[7] The microneedle preparation according to [6], wherein the amino acid is one or more selected from the group consisting of tyrosine, serine, threonine, phenylalanine, tryptophan, isoleucine, lysine, arginine, betaine, histidine, glutamine, glycine, and cysteine.
[8] The microneedle preparation according to any one of [1] to [7], wherein the microneedle preparation has a needle length of 50 to 350 um to be is inserted into epidermis.
[9] The microneedle preparation according to any one of [1] to [7], wherein the microneedle preparation has a needle length of 351 to 1000 um to be inserted into dermis.
[10] The microneedle preparation according to any one of [1] to [9], including a microneedle array containing a water-soluble polymer as a base and one or more selected from the group consisting of a lipase, a phosphatidylcholine, and deoxycholic acid.
[11] The microneedle preparation according to any one of [1] to [9], including a microneedle array containing a water-soluble polymer as a base and one or more selected from the group consisting of a lipase, a phosphatidylcholine, deoxycholic acid, tyrosine, L-carnitine, and caffeine.
[12] The microneedle preparation according to any one of [1] to [11], wherein the fat dissolving agent consists of a combination of a phosphatidylcholine and deoxycholic acid.
[13] The microneedle preparation according to any one of [1] to [12], further including an enzyme fermented plant extract.
[14] The microneedle preparation according to any one of [1] to [13], including a support film having an adhesive layer on one surface of the film on a surface opposite to a surface on which microneedles stand.
[15] The microneedle preparation according to [14], wherein the microneedle preparation includes a dissolvable microneedle array, and the adhesive layer contains a same component as the lipolytic agent, the fat dissolving agent, or the fat metabolism promoting agent contained in the dissolvable microneedle array at a concentration equal to or lower than a concentration in the microneedle array.
[16] The microneedle preparation according to any one of [1] to [15], wherein the microneedle preparation is for reducing an eye bag.

### EFFECT OF THE INVENTION

According to the present invention, it is possible to easily and uniformly dissolve subcutaneous fat.

### BRIEF DESCRIPTION OF THE DRAWINGS

FIG. 1 is a sectional view illustrating an example of a method for producing a microneedle array of the present invention.
FIG. 2A is a photograph of lower eyelids before the microneedle array in Test Example 2 is used.
FIG. 2B is a photograph of the lower eyelids after the microneedle array in Test Example 2 is used.
FIG. 3 is a graph showing a change in thickness of subcutaneous fat in Test Example 3.

### MODE FOR CARRYING OUT THE INVENTION

### Microneedle preparation (microneedle array)

A microneedle preparation of the present invention is a preparation including a microneedle array in which konide-shaped, pyramid-shaped, or needle-shaped microneedles having a height of 50 to 1000 um stand on a substrate having a thickness of 10 to 200 um.

The needle length is preferably 50 to 350 um when the microneedle preparation is to be inserted into epidermis.

The needle length is preferably 351 to 1,000 um when the microneedle preparation is to be inserted into dermis.

In the microneedles, a valuable substance is dispersed in a state of being dissolved or suspended in a base. The valuable substance may also be applied to the needle tips.

The microneedle preparation of the present invention includes at least one of a lipolytic agent, a fat dissolving agent, or a fat metabolism promoting agent as a valuable substance. The valuable substance is a component that acts on fat reduction and is contained in an amount of 0.1 mass% or more in the microneedle preparation. The microneedle preparation of the present invention is suitably used for the purpose of fat reduction and/or partial slenderizing and/or skin tightening. The content of the valuable substance is preferably 0.1 to 100 mass% and more preferably 0.2 to 80 mass% for decomposition, dissolution, or metabolism promotion of subcutaneous fat. The amount of the valuable substance per square cm is preferably 1 µg to 1,000 mg.

The microneedle array may contain a component for promoting fat combustion, a component for promoting decomposition and discharge of fat from adipocytes, a skin tightening component, and the like in combination with the valuable substance having decomposition, dissolution, and metabolism promotion action on fat to enhance the effect of decomposing and dissolving fat.

Examples of the lipolytic agent include lipolytic enzymes such as lipases, and hormones such as glucagon, adrenaline, noradrenaline, ghrelin, growth hormone, testosterone, and cortisol. It is also effective to use in combination a protease as a proteolytic enzyme, a collagenase as a collagenolytic enzyme, a hyaluronidase as a hyaluronan-degrading enzyme, and an enzyme fermented plant extract obtained by fermenting and extracting vegetables, fruits, and seaweed according to the care site and purpose.

Lipases are preferable as the lipolytic agent. When a lipase is used, a stabilizer of the lipase may coexist. Examples of the stabilizer include glycerol, glycine, mercaptoethanol, Triton X-100, Triton N-101, deoxycholic acid, Mg²⁺ ions, Ca²⁺ ions, BSA, and salts. Mg²⁺ ions and Ca²⁺ ions may coexist as a salt.

Examples of the fat dissolving agent include phosphatidylcholines, Fucus extracts, tyrosine, adenosine triphosphate and sodium salts thereof, Aesculus hippocastanum, Persian walnut, methylpropanediol, methylsilanol mannuronate, Pulsatilla cernua, Fumaria officinalis, deoxycholic acid, and chenodeoxycholic acid. Examples of the fat dissolving agent further include xanthine derivatives (caffeine, theophylline, theobromine, xanthine, aminophylline, choline theophylline, diprophyllin, proxyphylline, ocstriphyllin, etc.), Cocculus orbiculatus extract, thistle extract, Sinomenium acutum extract, Curcuma zedoaria extract, Fumaria officinalis extract, platycodon extract, Hedera rhombea extract, Piper nigrum extract, Cimicifuga simplex root extract, Centella asiatica extract, Gardenia jasminoides fruit extract, Fumaria officinalis, and strawberry leaf extract.

The fat dissolving agent is preferably one or more selected from the group consisting of a phosphatidylcholine, adenosine triphosphate and a sodium salt thereof, and deoxycholic acid. Among them, a fat dissolving agent consisting of a combination of phosphatidylcholine and deoxycholic acid is more preferable because the stability of phosphatidylcholine is improved.

Examples of the fat metabolism promoting agent include amino acids such as tyrosine, serine, threonine, phenylalanine, tryptophan, isoleucine, lysine, arginine, betaine, histidine, glutamine, glycine, and cysteine. Examples of a fat combustion promoting component include L-carnitine, inositol, capsaicin, caffeine, coenzyme Q10, and catechin.

The fat metabolism promoting agent is preferably one or more selected from the group consisting of an amino acid, L-carnitine, inositol, capsaicin, and caffeine, and it is also preferable to use one or more amino acids from the above.

For a non-dissolvable microneedle, the microneedle array of the present invention is produced by injection molding, press molding, or the like. The valuable substance is applied to the tips of the needles then dried to be used. As the base, plastics such as polyglycolic acid (PGA), polylactic acid-glycolic acid copolymers (PLGA), polyethylene terephthalate (PET), and polyethylene are convenient for molding.

In the present invention, a dissolvable microneedle array is more preferable. As the base, a water-soluble or biodegradable polymer substance is suitable, and examples thereof include sodium hyaluronate, hyaluronic acid oligomers and derivatives thereof, sodium chondroitin sulfate, hydroxypropyl cellulose, proteoglycan, gelatin, polyvinylpyrrolidone, hydrolyzed collagen, carboxymethyl cellulose, polyvinyl alcohol, dextran, and dextrin. It is simple to dissolve the valuable substance in the base, but the valuable substance may be applied to the needle tips.

The valuable substance and the base are essential components in the present invention, and other skin valuable substances may be added without limitation. Examples thereof include a pigmentation inhibitor, a moisturizer, a metabolic activator, an antioxidant, and an active oxygen scavenger/radical scavenger.

### Pigmentation inhibitor

A pigmentation inhibitor may be added to the present invention. Specific examples of the pigmentation inhibitor include p-aminobenzoic acid derivatives, salicylic acid derivatives, benzenesulfonamide derivatives, imidazole derivatives, naphthalene derivatives, hydroxyanthranilic acid or a salt thereof and derivatives thereof, anthranilic acid derivatives, coumarin derivatives, allantoin derivatives, nicotinic acid derivatives, ascorbic acid or a salt thereof and derivatives thereof, tocopherol or a salt thereof and derivatives thereof, tocotrienol or a salt thereof and derivatives thereof, kojic acid or a derivative thereof, oxybenzone, benzophenone, guaiazulene, shikonin, baicalin or a salt thereof and derivatives thereof, baicalein or a salt thereof and derivatives thereof, berberine or a salt thereof and derivatives thereof, apigenin or a salt thereof and derivatives thereof, luteolin or a salt thereof and derivatives thereof, kaempferol or a salt thereof and derivatives thereof, quercetin or a salt thereof and derivatives thereof, quercitrin or a salt thereof and derivatives thereof, isoquercitrin or a salt thereof and derivatives thereof, rutin or a salt thereof and derivatives thereof, myricetin or a salt thereof and derivatives thereof, naringenin or a salt thereof and derivatives thereof, hesperidin or a salt thereof and derivatives thereof, glutathione or a salt thereof and derivatives thereof, and ellagic acid or a salt thereof and derivatives thereof.

### Moisturizer

A moisturizer may be added to the present invention. Specific examples of the moisturizer include quince seed, agar or a derivative thereof, casein, glucose, galactose, mannose, xylose, fructose, maltose, isomaltose, cellobiose, genthiobiose, trehalose, pyrallose, 1,3-butylene glycol, glycerin, propylene glycol, polyethylene glycol, dipropylene glycol, 1,2-pentanediol, 1,5-pentanediol, 1,2-hexanediol, 1,6-hexanediol, mannitol and sorbitol, 1,2-propanediol, 1,3-propanediol, polypropylene glycol, 1,2-butanediol, 1,3-butanediol, 1,4-butanediol, pentylene glycol, hexylene glycol, 1,3-pentanediol, 1,4-pentanediol, erythritol, pentaerythritol, dipentaerythritol, xylitol, maltitol, inositol, panthenol or derivatives thereof, dextrin, gelatin, pectin, starch, carrageenan, carboxymethylchitin, carboxymethyl chitosan, chitosan salt, sulfated chitin, sulfated chitosan, phosphorylated chitin, phosphorylated chitosan, alginic acid or a salt thereof, hyaluronic acid or a salt thereof, chondroitin sulfate or a salt thereof, water-soluble polymers such as β-1,3-glucan, β-1,4-glucan, β-1,6-glucan, glucosamine, heparin, ethyl cellulose, methyl cellulose, carboxymethyl cellulose, carboxyethyl cellulose, sodium carboxyethyl cellulose, hydroxyethyl cellulose, hydroxypropyl cellulose, nitrocellulose, crystalline cellulose, hydroxypropyl methyl cellulose, polyvinyl alcohol, polyvinyl methyl ether, polyvinyl pyrrolidone, polyacrylate, carboxyvinyl polymer, dermatan sulfate, and keratan sulfate, pyrrolidone carboxylic acid or a salt thereof, polyglutamic acid or a salt thereof, pyrrolidone carboxylic acid contained in a natural moisturizing factor, urea, urocanic acid, betaine, sodium lactate, aspartic acid, glutamic acid, isoleucine, histidine, phenylalanine, threonine, serine, valine, proline, glycine, alanine, lysine, arginine, and ceramides such as ceramide 1, ceramide 2, ceramide 3, ceramide 4, ceramide 5, ceramide 6II, and ceramide 9.

### Metabolic activator

A metabolic activator may be added to the present invention. Specific examples of the metabolic activator include vitamin A group: retinol or a salt thereof and derivatives thereof, retinal or a salt thereof and derivatives thereof, dehydroretinal or a salt thereof and derivatives thereof, retinoic acid or a salt thereof and derivatives thereof, carotene or a salt thereof and derivatives thereof, lycopene or a salt thereof and derivatives thereof;
vitamin B group: thiamine or a salt thereof and derivatives thereof, riboflavin or a salt thereof and derivatives thereof, pyridoxine or a salt thereof and derivatives thereof, pyridoxal or a salt thereof and derivatives thereof, cyanocobalamin or a salt thereof and derivatives thereof, folic acid or a salt thereof and derivatives thereof, nicotinic acid or a salt thereof and derivatives thereof, pantothenic acid or a salt thereof and derivatives thereof, biotin or a salt thereof and derivatives thereof, choline or a salt thereof and derivatives thereof, inositol or a salt thereof and derivatives thereof;
vitamin C group: ascorbic acid or a salt thereof and derivatives thereof;
vitamin D group: ergocalciferol or a salt thereof and derivatives thereof, cholecalciferol and a salt thereof and derivatives thereof;
vitamin E group and the like: tocopherol or a salt thereof and derivatives thereof, tocotrienol or a salt thereof and derivatives thereof, ubiquinone or a salt thereof and derivatives thereof, linoleic acid or a salt thereof and derivatives thereof, linolenic acid or a salt thereof and derivatives thereof, arachidonic acid or a salt thereof and derivatives thereof, carnitine or a salt thereof and derivatives thereof, ferulic acid or a salt thereof and derivatives thereof, γ-oryzanol or a salt thereof and derivatives thereof;
vitamin P group: rutin or a salt thereof and derivatives thereof, hesperidin or a salt thereof and derivatives thereof, amino acids and the like: valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, histidine or a derivative thereof, amino acids such as sulfates, phosphates, nitrates, citrates, and an amino acid derivative of pyrrolidone carboxylic acid or the like, α-hydroxy acids such as glycolic acid, citric acid, malic acid, tartaric acid, lactic acid and succinic acid, 2-hydroxycarboxylic acids, polyhydroxycarboxylic acids, lactobionic acid, photosensitive element 301, hinokitiol, pantothenic acid or a derivative thereof, allantoin, trimethylglycine, and proteoglycan.

### Antioxidant

An antioxidant may be added to the present invention. Specific examples of the antioxidant include ascorbic acid or a salt thereof and derivatives thereof, tocopherol or a salt thereof and derivatives thereof, tocotrienol or a salt thereof and derivatives thereof, butylhydroxytoluene (BHT), butylhydroxyanisole (BHA), coenzyme Qn (n = 7 to 10), pyrroloquinoline quinone, propyl gallate, sesamol, and carotenoids.

### Active oxygen scavenger/radical scavenger

An active oxygen scavenger/radical scavenger may be added to the present invention. Specific examples of the active oxygen scavenger/radical scavenger include superoxide dismutase, catalase, glutathione peroxidase, bilirubin, quercetin, quercitrin, catechin, a catechin derivative, rutin or a derivative thereof, gallic acid or a salt thereof and derivatives thereof, curcumin or a salt thereof and derivatives thereof, transferrin, ceruloplasmin, coenzyme Qn (n = 7 to 10), uric acid, bilirubin, and metallothionein.

In addition to the components described above, components typically used for external preparations for skin such as cosmetics and pharmaceuticals may be added in the present invention. The additive component may be one or more selected from an aqueous component, an oily component, a plant extract, an animal extract, a powder, a surfactant, an oil agent, an alcohol, a pH adjusting agent, a preservative, a thickener, a pigment, a fragrance, and the like. These components may be part of the base, and they may also serve as valuable substances because of their effects on the skin (for example, skin tightening action).

The method for producing the microneedle array of the present invention is not particularly limited as long as the array is produced by any conventionally known method. Examples thereof include a method including casting a raw material aqueous solution obtained by dissolving or suspending the valuable substance in an aqueous solution or suspension composed of a base of the water-soluble polymer substance described above and adding other components as necessary, in a mold in which the shape of microneedles is formed, drying the solution, and then peeling off the obtained material. After peeling, the obtained material may be cut into a patch shape, and the patch may be lined with an adhesive support.

The above is a method for producing a dissolvable microneedle array. The microneedle array of the present invention is effective not only in a dissolvable type but also in a coating type microneedle array. In a production method of a coating type microneedle array, the microneedle array is prepared in advance by injection molding, press molding, or the like, and a mixture of a valuable substance and a base is applied to the needle tips of the array and dried.

The size of the patch (microneedle patch) formed of the microneedle array is 0.5 to 300 square cm. When the size is less than 0.5 square cm, the effect is limited, and it is difficult to exhibit the effectiveness. When it exceeds 300 square cm, a problem in adhesion is likely to occur in covering a body surface. To cover a wide body surface, a plurality of microneedle patches having a size of 300 square cm or less may be used. An appropriate needle length is 50 to 1000 um. A microneedle of 50 to 350 um acts on epidermis. A microneedle having a length larger than 350 um also acts on dermis.

To stably apply the microneedle patch to skin and hold the microneedle patch on the skin, it is not essential but more preferable that an adhesive support is provided on the back surface of the patch. The "back surface" refers to a surface opposite to a surface on which microneedles stand. The adhesive support is a support film having an adhesive layer on one surface.

As the adhesive layer in the present invention, an adhesive sheet made of a commercially available adhesive may be used. A rubber-based adhesive, a silicone-based adhesive, and the like may be used, and an acrylic adhesive is preferable in consideration of attachment to skin and adhesion to the support film. Specifically, the adhesive is a copolymer of acrylic acid alkyl esters and a copolymer mainly composed of an acrylic acid alkyl ester with acrylic acid, acrylamide, vinyl acetate, or the like. A plasticizer such as isopropyl myristate or isopropyl palmitate may be added to these adhesives to improve the adhesiveness to skin. The thickness of the adhesive layer is desirably 10 um or more and 300 um or less. Specifically, HiPAS (registered trademark) adhesive (acrylic ester, manufactured by CosMED Pharmaceutical Co., Ltd.) and MASCOS10 (product name) adhesive (acrylic ester, manufactured by CosMED Pharmaceutical Co., Ltd.) may be suitably used.

A low-molecular compound is often used as the fat dissolving agent that is a valuable substance in the present invention. In such a case, the fat dissolving agent contained in the microneedles may diffuse into the adhesive layer during a long-term storage period, resulting in a decrease in the content of the fat dissolving agent in the microneedles. Similar events may occur depending on the lipolytic agent and fat metabolism promoting agent to be used. To prevent such an event, it is effective from the viewpoint of storage stability to contain the same substance as the valuable substance impregnated into the microneedles also in the adhesive to prevent diffusion into the adhesive. The concentration of the fat dissolving agent, the lipolytic agent, or the fat metabolism promoting agent in the adhesive is desirably equal to or less than the concentration in the microneedle array.

The microneedle preparation, microneedle array, or microneedle patch of the present invention is applied to the skin from which fat is desired to be removed. The application site is the overall of skin and is not particularly limited. The number of sheets to be applied is set according to the area from which fat is desired to be removed. One application time is about 1 second to 24 hours, and in the case of multiple applications, it is effective to apply at intervals of 0 to 1 day. It is also effective to instantaneously dissolve the tips of the microneedles in the skin by supplying water from the back of the array immediately after application of the microneedle array to the skin to promote absorption of valuable substances into the skin. Such an application is an effective application method particularly in a beauty treatment or the like.

According to the present invention, partially slenderizing of a desired part of a body is possible. In particular, the effect is likely to be exhibited in an application to the face. For example, when the application is performed to a lower eyelid, an effect of reducing the bulge (eye bag) to the lower eyelid can be obtained.

### EXAMPLES

Hereinafter, the present invention will be described in more detail by the following Examples. These Examples are merely examples for specifically describing the present invention, and the scope of the present invention is not limited to these Examples.

### Example 1

### Production of microneedle array

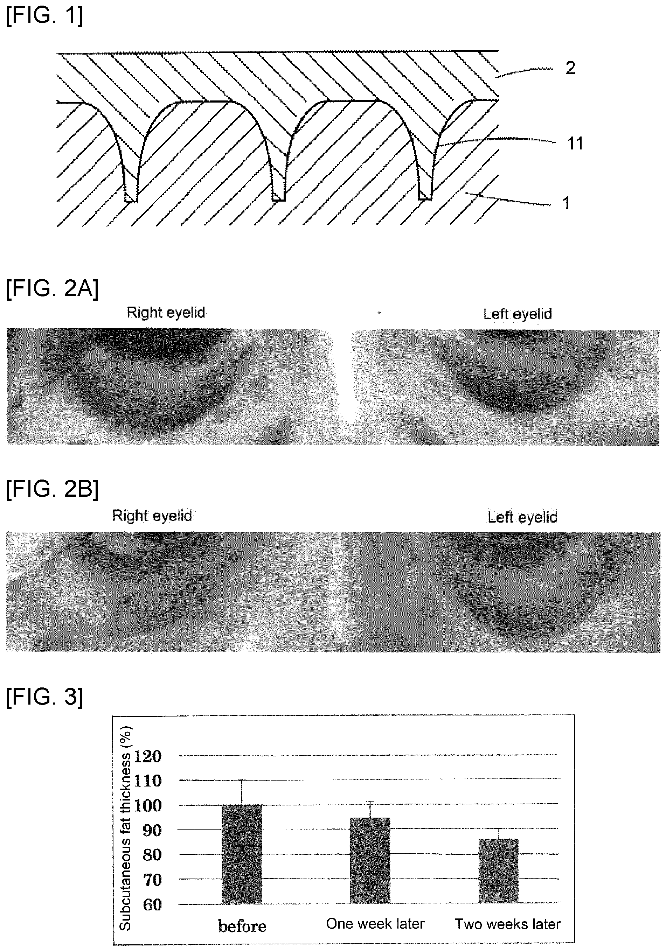

FIG. 1 is a sectional view illustrating an example of a method for producing a microneedle array of the present invention. In the drawing, reference numeral 1 denotes a mold in which a konide-shaped microneedle forming recess 11 is formed by transferring a konide-shaped microneedle pattern by electroforming, the konide-shaped microneedle pattern being formed by a lithography method of irradiating a photosensitive resin with light. Reference numeral 2 denotes a microneedle raw material liquid that is cast in the microneedle forming recess 11.

The microneedle forming recesses 11 are in a konide shape having a root diameter of 0.6 mm, a tip diameter of 0.02 mm, and a depth of 0.2 mm, which are arranged in a lattice pattern at intervals of 0.8 mm.

An aqueous solution (microneedle raw material solution) prepared by adding a phosphatidylcholine (Wako Pure Chemical Industries, Ltd.) and deoxycholic acid (Wako Pure Chemical Industries, Ltd.) dissolved to be 0.2 parts by mass and 0.8 parts by mass, respectively, in ethanol and glycerin to an aqueous solution in which 4 parts by mass of hyaluronic acid (product name "FCH-SU" manufactured by Kikkoman Biochemifa Company) and 0.1 parts by mass of a lipase (product name "Lipase AK Amano", manufactured by Wako Pure Chemical Industries, Ltd.) were dissolved in 100 parts by mass of water at room temperature were cast on the mold 1, and the moisture in the aqueous solution was evaporated. Thereafter, the obtained material was peeled off from the mold 1 and punched into an elliptical shape (10 x 50 mm, short diameter x long diameter).

### Production of microneedle array with protective adhesive tape

The elliptical microneedle array (10 x 50 mm, short diameter x long diameter) was set in the central part of a rectangular (16 x 60 mm) adhesive tape with a support having rounded corners, whereby a microneedle array with a protective adhesive tape of the present invention was obtained.

### Test Example 1 In vitro test

The microneedle array produced in Example 1 without a support was brought into close contact with the central part of the upper plane of beef tallow having a length of 2 cm, a width of 2 cm, and a thickness of 1 cm. Water in an amount of 40 mg was dropped from a dropper to the central part of the microneedle array to promote dissolution of the microneedle array. The upper surface of the beef tallow after being left to stand at room temperature for 20 hours was observed. The central part to which water was dropped was recessed in a lens shape, and the depth of the recess was about 1 mm at the deepest part. In this phenomenon, a decomposition component of beef tallow is generated by an oil decomposition/dissolution component blended in the microneedles, or a tallow-soluble component is generated by interaction with the dissolution component, and as a result of these products diffusing inside the beef tallow, a lens-shaped recess was generated.

### Comparative Test Example 1

The same test as Test Example 1 was performed except that the microneedle array brought into close contact in Test Example 1 was composed only of hyaluronic acid. The upper surface of the beef tallow did not change at all after being left to stand at room temperature for 20 hours.

### Test Example 2 In vivo test

A microneedle array produced in the same manner as in Example 1 except that no lipase was contained was applied to the lower right eyelid of an adult male volunteer before sleeping and peeled off in the morning of the next day. A test was performed in which the same application was performed for four days (four times in total). Nothing was applied to the lower left eyelid. Photographs of the lower eyelids before and after the test are shown in FIGS. 2A and 2B. It is clear that the bulge (eye bag) of the lower right eyelid is reduced as compared with the lower left eyelid.

### Example 2

### Production of microneedle array and protective adhesive tape for test

A microneedle array was produced in the same manner as in Example 1. The main composition of the array was 72 parts by mass of the hyaluronic acid, 5 parts by mass of the phosphatidylcholine, 15 parts by mass of the sodium deoxycholate, and 5 parts by mass of hydrolyzed collagen with respect to 100 parts by mass of the microneedle array. The produced microneedle array was punched into an elliptical shape in the same manner as in Example 1 and subjected to a test.

A rubber-based adhesive was used for the adhesive tape with a support, and the phosphatidylcholine and sodium deoxycholate were also contained in the adhesive to prevent transfer of the phosphatidylcholine and sodium deoxycholate from the array to the adhesive. Specifically, the adhesive (100 parts by mass) contained 1 part by mass of the phosphatidylcholine and 1 part by mass of the sodium deoxycholate in a basic composition composed of a styrene-isoprene resin, a tackifier resin, and oil. A microneedle array with a protective adhesive tape obtained in the same manner as in Example 1 had the same shape as in Example 1.

### Test Example 3

The microneedle array with a protective adhesive tape of Example 2 was applied to the cheeks of three adult female volunteers before sleeping and peeled off in the morning of the next day. A test in which the same application was performed three times/week was performed for two weeks, and the effectiveness of the microneedle array was evaluated. The subcutaneous fat thickness of the microneedle array application site before and after the test was measured using a subcutaneous fat thickness meter SR-803 (Tanita Corporation). The results are shown in Table 1 and FIG. 3. The principle of measuring the subcutaneous fat thickness is as follows. That is, fat hardly passes electricity, but muscle and intradermal water are conductive, and the thickness of subcutaneous fat is evaluated by measuring the electrical conductivity of the cheeks. In Table 1 and FIG. 3, the subcutaneous fat thickness before use is set to 100, and the values after use are shown. It is clear that the cheek subcutaneous fat was reduced by the application of the microneedle array.

**[Table 1]**

| Change in thickness of subcutaneous of cheek | | | | |
|---|---|---|---|---|
| Volunteer | Cheek | Before use | One week later | Two weeks later |
| A | Left | 100 | 85.7 | 80.0 |
| B | Right | 100 | 98.3 | 86.2 |
| C | Right | 100 | 95.2 | 90.3 |
| C | Left | 100 | 100.0 | 87.1 |
| Average | | **100** | **94.8±6.3** | **85.9±4.3** |

As shown by the above effectiveness test, the microneedle array exhibits a quantitative effect on the reduction of subcutaneous fat. There is no precedent for the microneedles and the patch having such efficacy.

### DESCRIPTION OF REFERENCE SYMBOLS

- 1: Mold
- 2: Microneedle raw material liquid
- 11: Microneedle forming recess

## Claims

1. A microneedle preparation comprising at least one of a lipolytic agent, a fat dissolving agent, or a fat metabolism promoting agent, the microneedle preparation being intended for fat reduction and/or partial slenderizing and/or skin tightening.

2. A microneedle preparation comprising 0.1 mass% or more of one or more components that act on fat reduction selected from the group consisting of a lipolytic agent, a fat dissolving agent, and a fat metabolism promoting agent, the microneedle preparation being intended for fat reduction and/or partial slenderizing and/or skin tightening.

3. The microneedle preparation according to claim 1 or 2, wherein the lipolytic agent is a lipase.

4. The microneedle preparation according to claim 3, comprising a lipase, a Mg salt, and a Ca salt.

5. The microneedle preparation according to claim 1 or 2, wherein the fat dissolving agent is one or more selected from the group consisting of a phosphatidylcholine, adenosine triphosphate and a sodium salt thereof, and deoxycholic acid.

6. The microneedle preparation according to claim 1 or 2, wherein the fat metabolism promoting agent is one or more selected from the group consisting of an amino acid, L-carnitine, inositol, capsaicin, and caffeine.

7. The microneedle preparation according to claim 6, wherein the amino acid is one or more selected from the group consisting of tyrosine, serine, threonine, phenylalanine, tryptophan, isoleucine, lysine, arginine, betaine, histidine, glutamine, glycine, and cysteine.

8. The microneedle preparation according to any one of claims 1 to 7, wherein the microneedle preparation has a needle length of 50 to 350 um to be is inserted into epidermis.

9. The microneedle preparation according to any one of claims 1 to 7, wherein the microneedle preparation has a needle length of 351 to 1000 um to be inserted into dermis.

10. The microneedle preparation according to any one of claims 1 to 9, comprising a microneedle array containing a water-soluble polymer as a base and one or more selected from the group consisting of a lipase, a phosphatidylcholine, and deoxycholic acid.

11. The microneedle preparation according to any one of claims 1 to 9, comprising a microneedle array containing a water-soluble polymer as a base and one or more selected from the group consisting of a lipase, a phosphatidylcholine, deoxycholic acid, tyrosine, L-carnitine, and caffeine.

12. The microneedle preparation according to any one of claims 1 to 11, wherein the fat dissolving agent consists of a combination of a phosphatidylcholine and deoxycholic acid.

13. The microneedle preparation according to any one of claims 1 to 12, further comprising an enzyme fermented plant extract.

14. The microneedle preparation according to any one of claims 1 to 13, comprising a support film having an adhesive layer on one surface of the film on a surface opposite to a surface on which microneedles stand.

15. The microneedle preparation according to claim 14, wherein the microneedle preparation includes a dissolvable microneedle array, and the adhesive layer contains a same component as the lipolytic agent, the fat dissolving agent, or the fat metabolism promoting agent contained in the dissolvable microneedle array at a concentration equal to or lower than a concentration in the microneedle array.

16. The microneedle preparation according to any one of claims 1 to 15, wherein the microneedle preparation is for reducing an eye bag.
